# EUROPEAN PATENT APPLICATION

(11) **EP 4 806 954 A1**
(43) Date of publication of application: **16.09.2026**
(21) Application number: 25386017.5
(22) Date of filing: 12.03.2025
(51) Int. Cl.: C09B 23/01, C09B 23/14, A61B 5/00, C07H 1/00, C12Q 1/6876

(54) **DICYANOMETHYLENE HETEROCYCLIC COMPOUNDS**

(71) Applicant: National and Kapodistrian University of Athens, 10679 Athens (GR); University of Ioannina - Special Account For Research Funds (Elke), 45110 Ioannina (GR); Tzakos, Andreas, 45500 Ioannina (GR); Diamandis, Dimitrios, 45221 Ioannina (GR); Kyrkou, Stavroula, 45560 Ioannina (GR)
(72) Inventor: Trougakos, Ioannis, 15701 Athens (GR); Sklirou, Aimilia, 15701 Athens (GR); Tzakos, Eleftherios Paraskevas, 15701 Athens (GR); Tzakos, Andreas, 45500 Ioannina (GR); Diamandis, Dimitrios, 45221 Ioannina (GR); Kyrkou, Stavroula, 45560 Ioannina (GR)
(74) Representative: Dodou, Evdokia

(57) **Abstract**

A compound according to Formula (I) or a salt thereof and the use of the compound or a salt thereof for the detection of senescent cells in a biological sample.

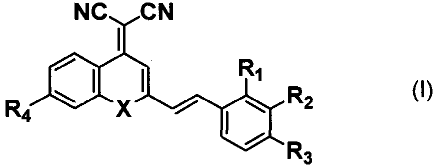

## Description

### Field of the invention

The present invention relates to new dicyanomethylene heterocycles and their use as senolytics.

### Background of the invention

Aging is a time-dependent procedure that is characterized by the progressive loss of physical function and organismal homeodynamics. It is the major risk factor for numerous diseases including type 2 diabetes, cardiovascular diseases, cancer, neurodegeneration and other vulnerabilities. The major hallmarks of aging, among others, include epigenetic modifications, genomic instability, telomere attrition, mitochondrial dysfunction as well as increasing rates of cellular senescence. Cellular senescence can be triggered by either telomere shortening (replicative senescence, RS) or in normal cells with long telomeres as a response to various damaging stimuli (stress-induced premature senescence, SIPS) including DNA damage, reactive oxygen species, aberrant activation of oncogenes, etc. Whereas cellular senescence (RS or SIPS), a state of highly stable cell cycle arrest, is considered an intrinsic tumor suppression mechanism, it appears that age-related accumulation of senescent cells in the human body can promote tumorigenesis e.g., via their senescence-associated secretory phenotype (SASP); a phenotype where senescent cells secrete high levels of immune modulators, inflammatory cytokines and proteases promoting thus tumor growth (Gorgoulis, V. et al. Cellular Senescence: Defining a Path Forward. Cell 179, 813-827, doi:10.1016/j.cell.2019.10.005 (2019)). Therefore, although cellular senescence is an adaptation "aiming" to interrupt the proliferation of damaged and/or stressed cells, the gradual accumulation of senescent cells in aged tissues leads to tissue/organ dysfunction and accelerates aging (Di Micco, R., Krizhanovsky, V., Baker, D. & d'Adda di Fagagna, F. Cellular senescence in ageing: from mechanisms to therapeutic opportunities. Nat Rev Mol Cell Biol 22, 75-95, doi:10.1038/s41580-020-00314-w (2021)). A great number of biomarkers and phenotypic characteristics have been developed to monitor cellular senescence including measurement of SASP-related cytokines, the expression of cell cycle inhibitors and/or tumor suppressors (e.g., p16, p21 or p53), as well as the detection of β-galactosidase in senescent cells (Kumari, R. & Jat, P. Mechanisms of Cellular Senescence: Cell Cycle Arrest and Senescence Associated Secretory Phenotype. Front Cell Dev Biol 9, 645593, doi:10.3389/fcell.2021.645593 (2021)).

B-galactosidase (β-gal) is a lysosomal hydrolase that catalyzes the cleavage of the galactose moiety and converts the lactose into monosaccharide. Its optimal enzymatic activity in young replicating human cells ranges from pH 4 to 4.5; yet, by using certain biochemical reactions at pH 6 an β-galactosidase isoform that accumulates specifically in senescent (RS or SIPS) cells can be used to discriminate these cells from young replicating human cells (Lee, B. Y. et al. Senescence-associated beta-galactosidase is lysosomal beta-galactosidase. Aging Cell 5, 187-195, doi:10.1111/j.1474-9726.2006.00199.x (2006)). It is not thus surprising that in order to monitor β-gal activity in different physiological conditions, including senescence and aging, or cancer, a great number of diagnostics have been developed (Trifonov, S., Yamashita, Y., Kase, M., Maruyama, M. & Sugimoto, T. Overview and assessment of the histochemical methods and reagents for the detection of β-galactosidase activity in transgenic animals. Anat Sci Int 91, 56-67, doi:10.1007/s12565-015-0300-3 (2016)). Recently, optical imaging has gained increasing attention as a non-invasive to tissue technique characterized by its high sensitivity and low cost. Nowadays, a plethora of fluorescent probes have been developed to visualize β-gal in cells (Safir Filho, M., Dao, P., Gesson, M., Martin, A. R. & Benhida, R. Development of highly sensitive fluorescent probes for the detection of β-galactosidase activity - application to the real-time monitoring of senescence in live cells. Analyst 143, 2680-2688, doi:10.1039/c8an00516h (2018)). However, a relatively small number of these probes discriminate these cells from young replicating human cells. Furthermore, a relatively small number of these probes emit in the near infrared region (650-900 nm), that permits deep tissue penetration imaging and annihilation of autofluorescence interference. As cellular senescence depends on the detection of the enzymatic activity of senescence-associated beta-galactosidase (SA-β-gal), which is overexpressed and accumulates in lysosomes, a lack of near-infrared (NIR) fluorescent probes that target this specific subcellular organelle occurs (Gao, Y. et al. Two-Dimensional Design Strategy to Construct Smart Fluorescent Probes for the Precise Tracking of Senescence. Angewandte Chemie International Edition 60, 10756-10765, doi:https://doi.org/10.1002/anie.202101278 (2021)). Therefore, there is a need for the development of NIR fluorescent probes suitable for the detection of the enzymatic activity of SA-β-gal in lysosomes.

### Summary of the invention

The present invention provides a compound or Formula (I), or a salt thereof 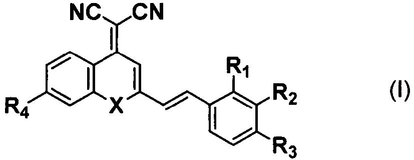 wherein
- X is selected from the group consisting of O, S, Se, -N-(CH₂)ₙ-CH₃;
- each one of R₁, R₂ and R₃ is selected from the group consisting of
   H, halogen, 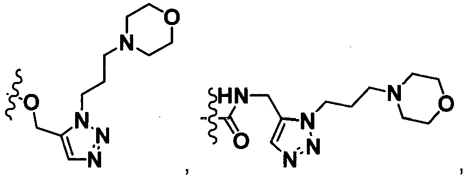 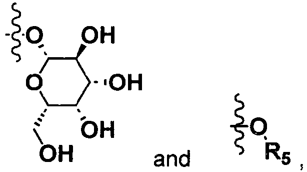 provided that
   a) one of R₁, R₂ and R₃ is 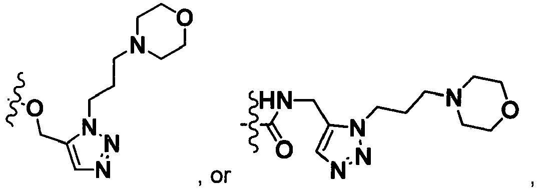 and
   b) one of R₁, R₂ and R₃ is 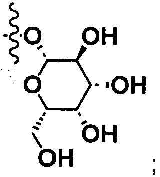
- R₄ is H, or 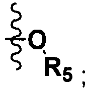
- R₅ is H, or -(CH₂)ₙ-CH₃
- n is 0, 1, 2, or 3.

The compounds of the present invention can act as colorimetric and fluorogenic probes that emit in the NIR region. Furthermore, the compounds of the present invention can accumulate in lysosomes and can recognize SA-β-gal.

The present invention provides also the use of a compound as defined above for the detection of senescent cells.

### Brief description of the drawings

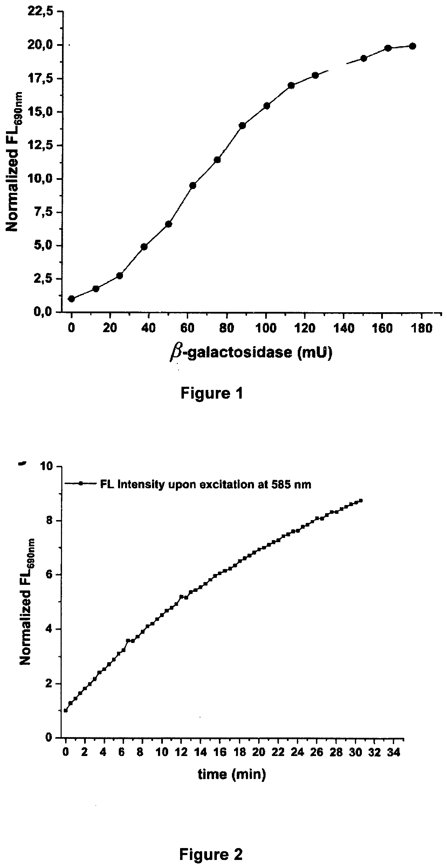

Figure 1 shows spectral properties of a compound of the present invention (10 µM) in DMSO: phosphate buffer (PB) (3:7 v/v, 10 mM, pH 6.00). The figure shows, the Normalized Fluorescent intensity of a compound of the present invention upon excitation at 585 nm in the presence of different concentrations of β-galactosidase.
Figure 2 shows the time dependence of emission spectra (0-30 min) of a compound of the present invention in DMSO: PB (3:7 v/v, 10 mM, pH 6.00) upon excitation at 585 nm following treatment with 0.1 U of *β*-galactosidase.

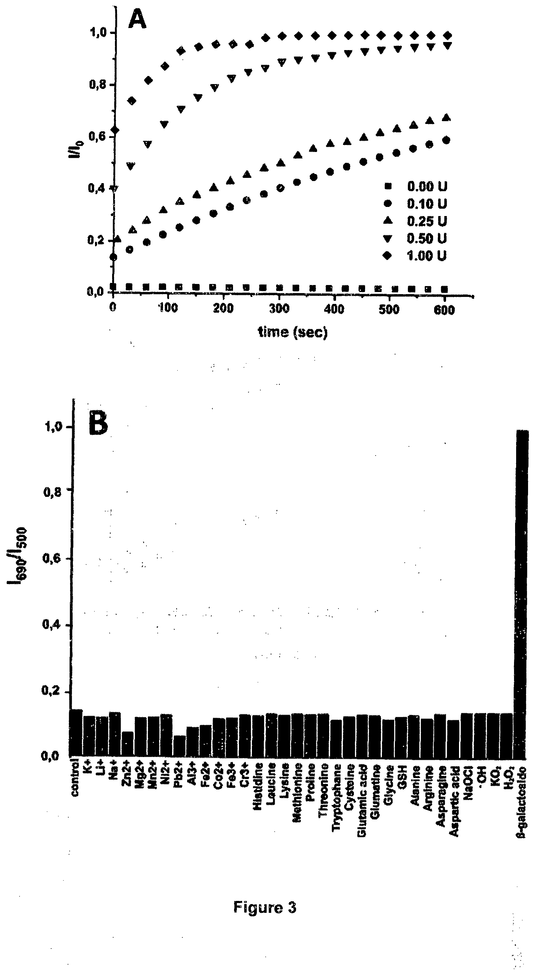

Figure 3 shows (A) Kinetics of the fluorescence signal at 690 nm of a compound of the present invention (10 µM) in DMSO: PB (3:7 v/v, 10 mM, pH 6.00) upon excitation at 585 nm in the absence or presence of different *β*-galactosidase concentrations. (B) Fluorescence ratio (*I*₆₉₀ /*I*₅₀₀) responses of a compound of the present invention (10 µM) to various analytes in DMSO: PB (3:7 v/v, 10 mM, pH 6.00) upon excitation at 465 nm.

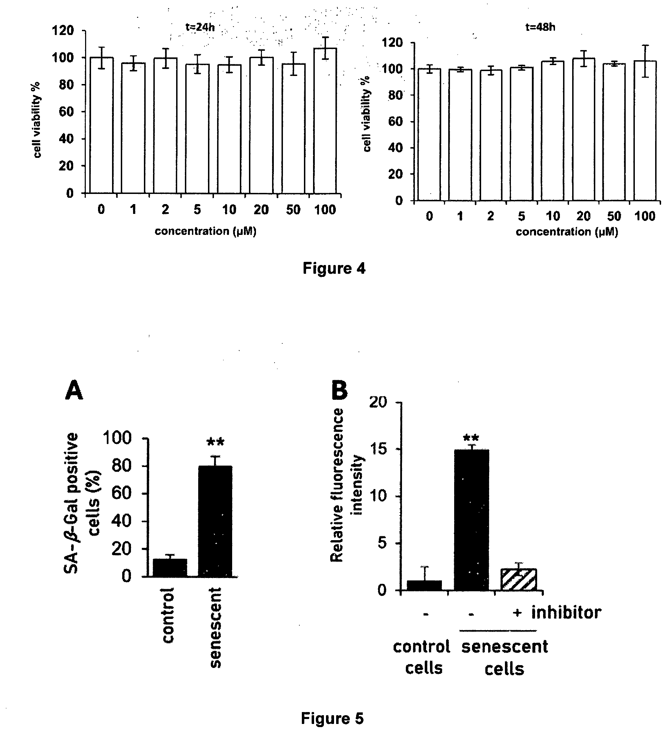

Figure 4 shows the cytotoxic effect of a compound of the present invention in normal human skin fibroblasts. Relative (%) survival levels (MTT assay) of BJ skin fibroblasts incubated with the indicated concentrations of a compound of the present invention for 24 h (A) and 48 h (B). Control samples values were set to 100%; bars, ± SD (n ≥ 2).
Figure 5 shows (A) relative (%) number of SA-β-gal positive cells following SA-β-gal cytochemical staining of control BJ cells or cells treated (three exposures of 48 h each) with 300 µM H₂O₂ (B) quantitation of intensity of compound fluorescence in control and premature senescent (H₂O₂ treated) cells. Cells were cultured on coverslips and incubated with 10 µM of a compound of the present invention for 1h, upon or without pretreatment with the β-gal inhibitor (D-galactose; 1 mM) for 1 h. Cells were then fixed, stained with DAPI and mounted on slides before observation under CLSM following excitation at 543 nm. Bars ± SD. ** p < 0.01.

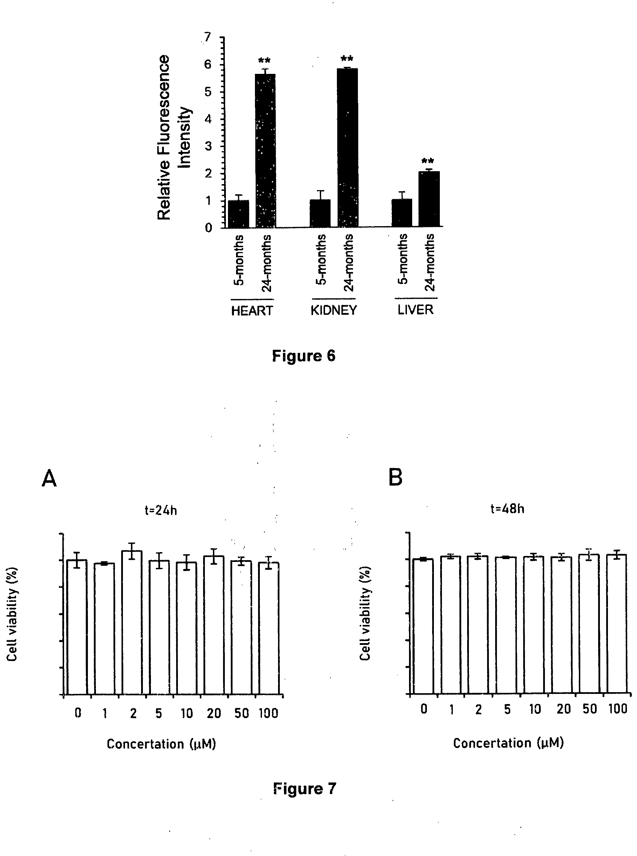

Figure 6 shows the relative (%) fluorescence of a compound of the present invention (20 µM) in tissue sections from 5-, and 24-month-old C57BL/6J male mice. Control (5 months old) samples values were set to 1. P-value was calculated using unpaired t-test. Bars, ± SD; * *p <* 0.05; ** *p <* 0.01.
Figure 7 shows the cytotoxic effect of a compound of the present invention in human normal skin fibroblasts. Relative (%) survival levels (MTT assay) of BJ skin fibroblasts incubated with the indicated concentrations of a compound of the present invention, for 24 h (A) and 48 h (B). Control samples values were set to 100%; bars, ± SD (n ≥ 2).

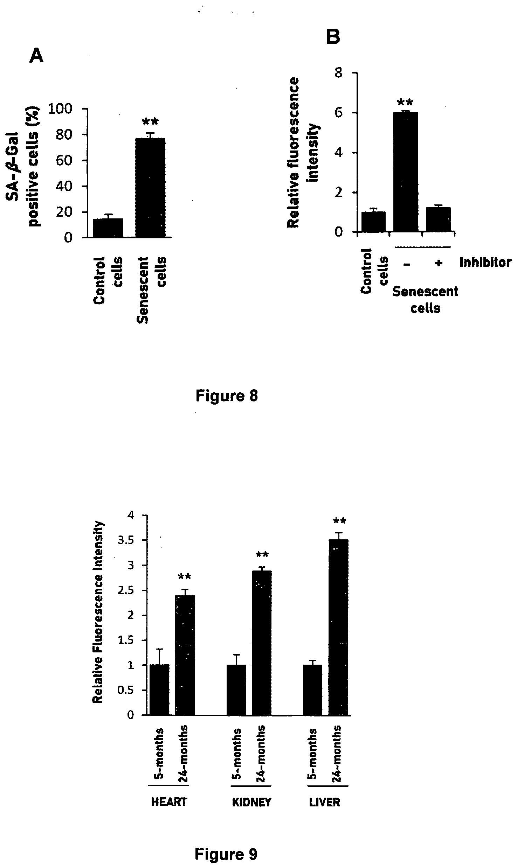

Figure 8 shows (A) relative (%) number of SA-β-gal positive cells following SA-β-gal cytochemical staining of control BJ cells or cells treated (three exposures of 48 h each) with 300 µM H₂O₂ (B) quantitation of intensity of compound fluorescence in control and premature senescent (H₂O₂ treated) cells. Cells were cultured on coverslips and incubated with 10 µM of a compound of the present inventoin for 1h, upon or without pretreatment with the *β*-gal inhibitor (D-galactose; 1 mM) for 1 h. Cells were then fixed, stained with DAPI and mounted on slides before observation at CLSM following excitation at 543 nm. Bars ± SD. ** *p <* 0.01.
Figure 9 shows the relative (%) fluorescence of a compound of the present invention (20 µM) in tissue sections from 5-, and 24-month-old C57BL/6J male mice. Control (5 months old) samples values were set to 1. *P*-value was calculated using unpaired *t*-test. Bars, ± SD; * *p <* 0.05; ** *p <* 0.01.

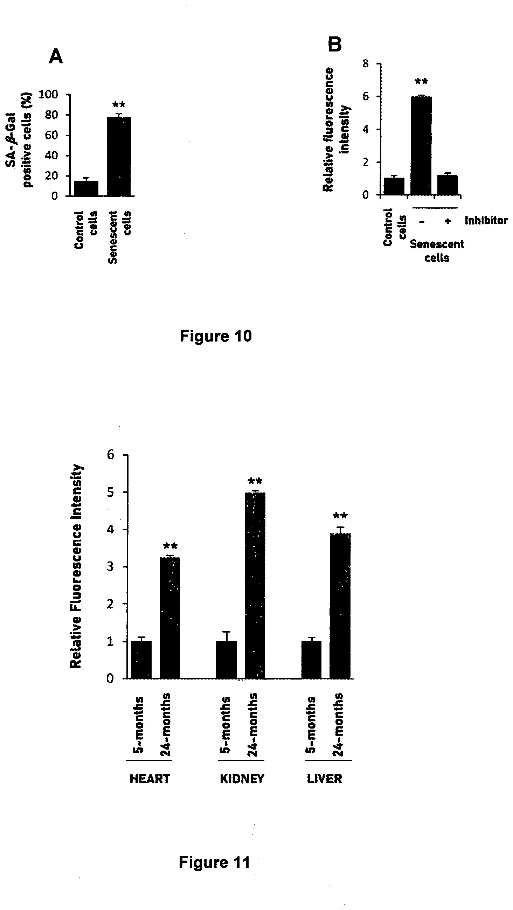

Figure 10 shows (A) relative (%) number of SA-β-gal positive cells following SA-β-gal cytochemical staining of control BJ cells or cells treated (three exposures of 48 h each) with 300 µM H₂O₂ (B) quantitation of intensity of compound fluorescence in control and premature senescent (H₂O₂ treated) cells. Cells were cultured on coverslips and incubated with 5 µM of a compound of the present invention for 1h, upon or without pretreatment with the *β*-gal inhibitor (D-galactose; 1 mM) for 1 h. Cells were then fixed, stained with DAPI and mounted on slides before observation under a CLSM following excitation at 543 nm. Bars ± SD. ** *p* < 0.01.
Figure 11 shows the relative (%) fluorescence of a compound of the present invention (20 µM) in tissue sections from 5-, and 24-month-old C57BL/6J male mice. Control (5months old) samples values were set to 1. *P*-value was calculated using unpaired *t-*test. Bars, ± SD; * *p <* 0.05; ** *p* < 0.01.

### Detailed description of the invention

The present invention provides a compound or Formula (I), or a salt thereof 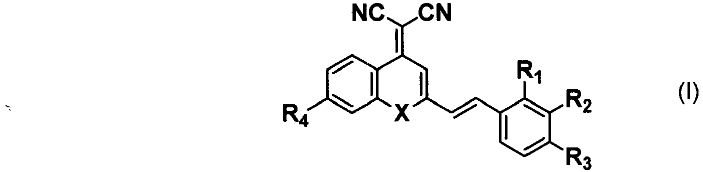 wherein
- X is selected from the group consisting of O, S, Se, -N-(CH₂)ₙ-CH₃;
- each one of R₁, R₂ and R₃ is selected from the group consisting of
   H, halogen, 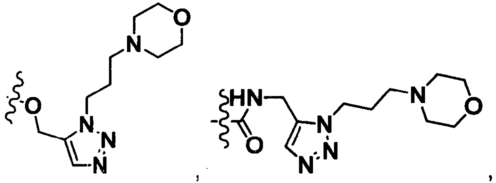 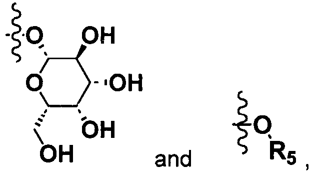 provided that
   a) one of R₁, R₂ and R₃ is 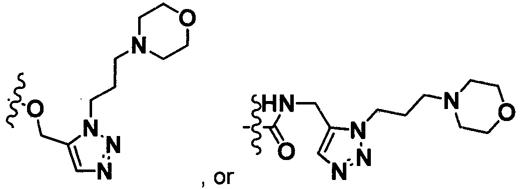 and
   b) one of R₁, R₂ and R₃ is 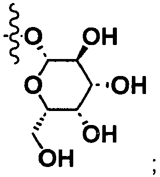
- R₄ is H, or 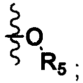
- R₅ is H, or -(CH₂)ₙ-CH₃;
- n is 0, 1, 2, or 3.

Preferably, the halogen is selected from the group consisting of Cl, Br, and I.

Preferably, X is selected from the group consisting of O, S and Se.

Preferably, R₁ or R₃ is

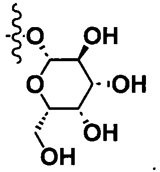

According to a preferred embodiment,
R₃ is 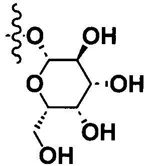 and
X is selected from the group consisting of O, S and Se.

According to another preferred embodiment,
R₁ is 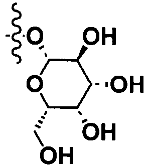 and
X is selected from the group consisting of O, S and Se.

A general route of synthesis of the compounds of the present invention is shown in Scheme 1.

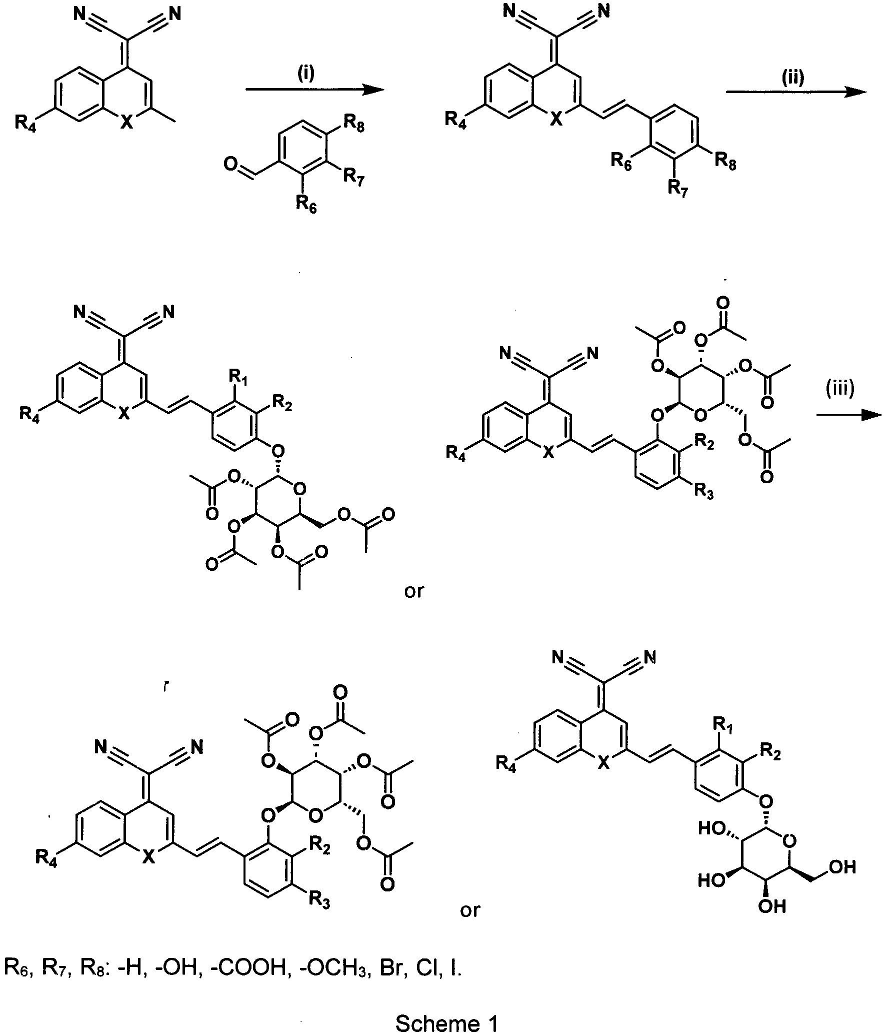

A dicyanomethylene-4H-pyran (DCM) fluorophore can undergo a condensation reaction (reaction (i) in Scheme 1) with a variety of substituted benzaldehydes. This reaction is typically carried out in polar aprotic solvents, such as acetonitrile, with a mild organic base added in catalytic amounts. Alternatively, it can proceed in polar protic solvents, such as methanol or ethanol, in the presence or absence of a catalytic amount of organic base. The condensation reaction is typically carried out under reflux conditions, for example, for 4 to 24 hours.

When the substituted benzaldehyde contains a free hydroxyl group, the D-galactopyranosyl moiety can be introduced into the fluorophore structure. To achieve glycosylation (reaction (ii) in Scheme 1), an appropriate derivative, such as tetra-O-acetyl-α-D-galactopyranosyl-1-bromide, and a mild organic base in a suitable solvent, such as a polar, non-protic solvent could be utilized. The reaction typically occurs at room temperature and lasts, for example, from 6 to 24 hours.

In the case where sugar moiety is in the R₂ position (not shown in Scheme 1), the synthetic pathway can proceed as follows:
A) If the R₇ position is an -OH group and the sugar contains a halogen at the anomeric position, nucleophilic aromatic substitution can occur in the presence of a mild base (i.e. triethylamine (TEA)) and a polar protic or aprotic solvent.
B) Similarly, if the R₇ position is occupied by a halogen and the anomeric position of the sugar is an -OH group (with the remaining hydroxyl groups been protected, for example with a tert-butyloxycarbonyl (Boc) group), the substitution can proceed under the same conditions.

If the R₇ position is a hydroxyl group and the anomeric position of the sugar is also a hydroxyl group, coupling via a carbonate bond can be achieved, for example, using bis(4-nitrophenyl) carbonate and a mild base in an organic aprotic solvent.

Following substitution, the final step involves deprotection of the sugar moiety, (reaction (iii) in Scheme 1), such as the removal of acetyl groups. The deprotection reaction can be performed for example, using sodium methoxide in methanol, under ambient conditions, and typically completes within 1 to 8 hours.

The compounds of the present invention contain basic groups and can form base addition salts. Examples of base addition salts include, sodium, potassium, calcium, magnesium, ammonium, choline, diethylamine, 2-diethylaminoethanol, N,N-dimethylethanolamine, tromethamine, ethanolamine, lysine and arginine salts. A base addition salt can be prepared following methods well known in the art. For example, a compound of the present invention can be combined with a base in a suitable solvent, such as acetone, toluene pyridine, or dichloromethane and the mixture is stirred, typically under heating until the corresponding salt is formed.

The compounds of the present invention selectively accumulate in lysosomes, fluoresce in the NIR region and recognize SA-β-gal. Specifically, the compounds of the present invention accumulate in lysosomes through a morpholine analogue; emit near-infrared light through a fluorescent probe and modify the fluorescence's intensity to longer wavelengths through a α-D-galactopyranosyl moiety that is recognized by the SA-β-gal enzyme.

The compounds of the present invention are responsive to the elevated levels of SA-β-gal that is overexpressed in senescent cells through a colorimetric bathochromic 160 nm shift in the UV-Vis spectrum and a parallel 20-fold enhancement of the fluorescent signal at 690 nm.

The present invention provides also the use of a compound as defined above for the detection of senescent cells in a biological sample. Preferably, the biological sample is obtained from a human subject.

According to an embodiment of the present invention, the detection of senescent cells is carried out *in vitro,* in cells obtained from a subject, such as a human. The cells are, for example, incubated with an aqueous solution of a compound of the present invention for one hour under physiological conditions (5% CO₂, 95% humidity, 37 °C). Following incubation, the cells are fixed with a 4% formaldehyde aqueous solution and analyzed using confocal microscopy. Visualization can be achieved upon excitation of the probe at 543 nm, using a confocal laser scanning microscope, equipped with a high-resolution objective and an acquisition and analysis software. Fluorescence intensity quantification can be conducted on captured images using imaging software.

According to another embodiment of the present invention, the detection of senescent cells is carried out *ex vivo,* in a tissue sample obtained from a subject, such as a human. For example, the tissue is washed with phosphate-buffered saline and frozen in liquid nitrogen. The frozen tissue is then sectioned at -20 °C using a cryo-cut microtome. A section of the tissue is then incubated with a solution of a compounds of the present invention for one hour at room temperature, fixed with a 4% formaldehyde aqueous solution, and subsequently analyzed via high resolution confocal microscopy. Visualization can be achieved upon excitation of the probe at 543 nm, using a confocal laser scanning microscope, equipped with a high-resolution objective and an acquisition and analysis software. Fluorescence intensity quantification can be conducted on captured images using imaging software.

### Examples

### Example 1

Scheme 2 shows the synthesis of compound 1, which is a compound according to the present invention.

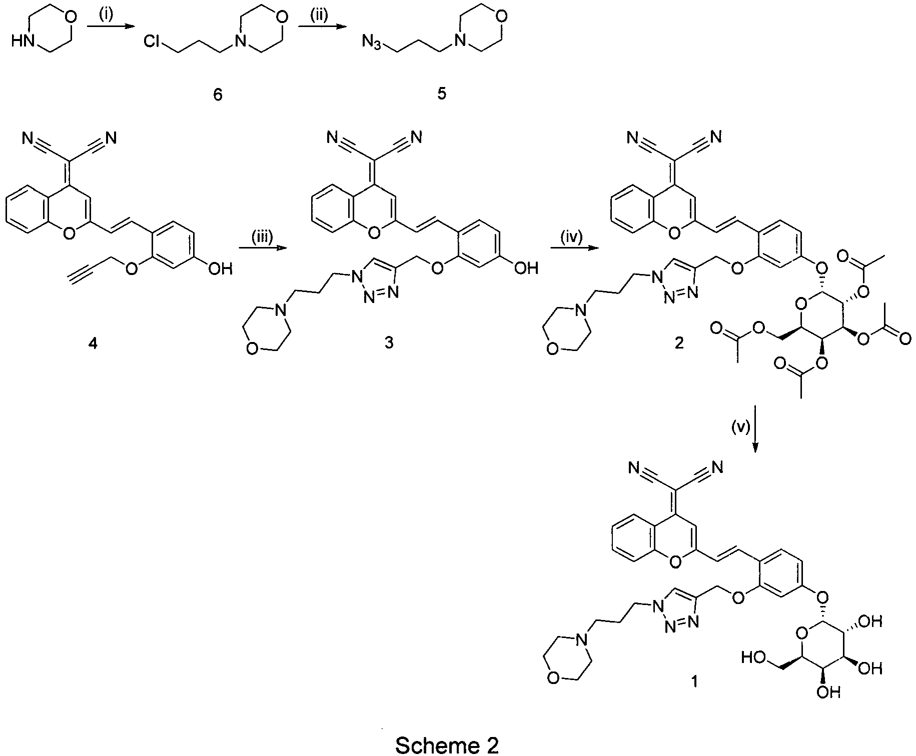

### Synthesis of compound 6

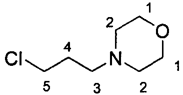

To a stirred solution of morpholine (110 µL, 1.26 mmol), in toluene (2 mL), 3-iodo 1-chloro propane (135 µL, 1.26 mmol) was added and the reaction mixture was refluxed for 4 hours. After cooling to room temperature, 5% NaOH solution was added to the reaction mixture and the residue was extracted twice with toluene. The organic layer was dried over Na₂SO₄ and concentrated under reduced pressure to afford a vicious yellow oil (90 mg, 43.5%).¹H NMR (400 MHz, CDCl₃) δ: 3.72-3.68 (t, *J* = 4.1 Hz, 4H, H-1), 3.36-3.30 (t, *J* = 6.7 Hz, 2H, H-3), 2.50-2.41 (m, 6H, H-2 & H-5), 1.98-1.90 (q, 2H, H-4).¹³C NMR (100 MHz, CDCl₃) δ: 66.92 (C-1), 55.62 (C-3), 53.66 (C-2), 42.88 (C-5), 29.51 (C-4).

### Synthesis of compound 5

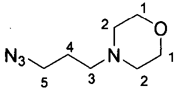

To a stirred solution of compound **6** (70 mg, 0.428 mmol), in a mixture of H₂O / MeOH (1:5, 1 mL / 5 mL), sodium azide (140 mg, 2.13 mmol) was added, and the reaction mixture was refluxed overnight. Next, the solvent was removed under reduced pressure, the residue was diluted and extracted twice with ethyl acetate to afford the desired compound as a yellow oil (62 mg, 85%).¹H NMR (400 MHz, CDCl₃) δ: 3.71-3.64 (t, *J =* 4.1 Hz, 4H, H-1), 3.35-3.30 (t, *J* = 6.7 Hz, 2H, H-3), 2.43-2.36 (m, 6H, H-2 & H-5), 1.78-1.69 (q, 2H, H-4).¹³C NMR (100 MHz, CDCl₃) δ: 66.91 (C-1), 55.61 (C-3), 53.61 (C-2), 49.45 (C-5), 25.91 (C-4).

### Synthesis of compound 3

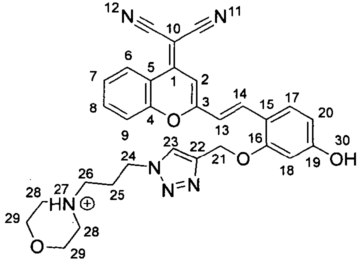

To a vigorous solution of compound **4** (60.3 mg, 0.164 mmol, compound 4 was synthesized according to Diamantis, Dimitrios A et al. "Biotin-Yellow a biotin guided NIR turn-on fluorescent probe for cancer targeted diagnosis",¹⁴) in dimethylformamide (DMF, 5 mL), - sodium ascorbate (6.5 mg, 0.033 mmol), compound **5** (30 mg, 0.164 mmol) and CuSO₄*5H₂O (3.06 mg, 0.012 mmol) were added and the reaction mixture was stirred for 16 hours. After TLC analysis had shown the full conversion of compound 3, the reaction mixture was concentrated under reduced pressure and the residue was purified by HPLC (40:60 H₂O: ACN to 0:100 H₂O: ACN, 254 nm) to afford a crimson solid (55 mg, 62.5%).¹H NMR (500 MHz, DMSO-d₆) δ: 10.37 (s, 1H, OH-30), 9.76 (br, 1H, NH-27), 8.73-8.67 (dd, *J* = 8.4 Hz & *J* = 1.3 Hz, 1H, H-6), 8.33 (s, 1H, H-23), 7.91-7.88 (t, *J* = 7.2 Hz, 1H, H-8), 7.87-7.81 (d, *J* = 16.1 Hz, 1H, H-14), 7.74-7.71 (dd, *J* = 8.5 Hz & *J* = 1.2 Hz, 1H, H-17), 7.66-7.62 (d, *J* = 8.5 Hz, 1H, H-9), 7.62-7.58 (td, *J* = 7.71 Hz & *J =* 1.0 Hz, 1H, H-7), 7.25-7.19 (d, *J* = 16 Hz, 1H, H-13), 6.83 (s, 1H, H-2), 6.73-6.70 (d, *J =* 2.1 Hz, 1H, H-18), 6.53-6.48 (dd, *J* = 8.4 Hz & *J* = 1.6Hz, 1H, H-20), 5.29 (s, 1H, H-21), 4.53-4.44 (t, *J* = 7.1 Hz, 2H, H-24), 4.01-3.88 (br, 2H, H-29), 3.68-3.54 (br, 2H, H-29), 3.19-3.10 (m, 2H, H-26), 3.10- 2.96 (br, 2H, H-28), 2.31-2.23 (q, 2H, H-25).¹³C NMR (125 Hz, DMSO-d₆) δ: 162.26 (C-19), 159.72 (C-16), 152.83 (C-4), 143.31 (C-22), 136.24 (C-8), 134.82 (C-14), 131.13 (C-9), 126.64 (C-7), 125.43 (C-6), 125.32 (C-23), 119.41 (C-17), 119.44 (C-5), 117.72 (C-12), 116.85 (C-11), 116.23 (C-13), 109.61 (C-20), 106.23 (C-2), 101.43 (C-18), 64.24 (C-29), 62.42 (C-21), 53.63 (C-26), 51.75 (C-28), 24.51 (C-25).

### Synthesis of compound 2

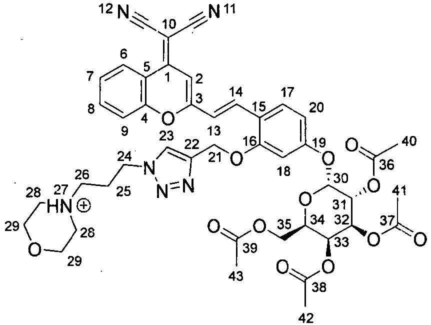

In a chilled solution of compound 3 (60 mg, 0.112 mmol), in dry acetonitrile (5 mL), TEA (141.68 µL, 1.12 mmol) was added, the reaction mixture was stirred for 5 minutes followed by the addition of tetra-O-acetyl-α-D-galactopyranosyl-1-bromide (184.2 mg, 0.448 mmol) and the reaction mixture was stirred at room temperature under nitrogen atmosphere for 15 hours. The solvent was removed under reduced pressure, and the residue was purified by HPLC (30:70 H₂O: ACN to 0: 100 H₂O: ACN, 254 nm) to afford the desired compound as brown solid (42 mg, 43.2 %).¹H NMR (400 MHz, DMSO-d₆) δ: 9.75 (br, 1H, NH-27), 8.75-8.72 (d, *J* = 8.3 Hz, 1H, H-6), 8.36 (s, 1H, H-23), 7.96-7.88 (t, *J =* 7.6 Hz, 1H, H-8), 7.87-7.82 (d, *J* = 16.2 Hz, 1H, H-14), 7.82-7.79 (d, *J* = 8.7 Hz, 1H, H-17), 7.78-7.72 (d, *J* = 8.4 Hz, 1H, H-9), 7.67-7.60 (t, *J* = 7.4 Hz, 1H, H-7), 7.41-7.34 (d, *J* = 16Hz, 1H, H-13), 6.98-6.94 (d, *J* = 2.1 Hz, 1H, H-18), 6.92 (s, 1H, H-2), 6.76-6.73 (dd, *J* = 8.4Hz & *J* = 2.1 Hz, 1H, H-20), 5.68-5.64 (d, *J* = 7.0 Hz, 1H, H-30), 5.40-5.34 (m, 3H, H-21 & H-32), 5.31-5.21 (m, 2H, H-31, H-33), 4.55-4.47 (m, 3H, H-24 & H-34), 4.17-4.09 (m, 2H, H-35), 4.04-3.91 (m, 2H, H-29), 3.69-3.57 (m, 2H, H-29), 3.23-3.12 (m, 2H, H-26), 3.11- 2.96 (br, 2H, H-28), 2.31-2.22 (q, 2H, H-25), 2.16 (s, 3H, H-43), 2.06 (s, 3H, H-42), 2.00 (s, 3H, H-41), 1.96 (s, 3H, H-40).¹³C NMR (100 MHz, DMSO-d₆) δ: 170.42 (C-39), 170.34 (C-38), 170.03 (C-37), 169.73 (C-36), 159.78 (C-3), 159.13 (C-16), 158.49 (C-4), 153.36 (C-19), 152.46 (C-9), 142.89 (C-22), 135.85 (C-8), 133.59 (C-17), 130.57 (C-14), 126.69 (C-7), 125.20 (C-23 & C-6), 119.48 (C-5), 118.78 (C-15), 117.73 (C-13), 117.63 (C-12), 116.44 (C-11), 109.23 (C-20), 106.71 (C-2), 102.85 (C-18), 97.48 (C-30), 71.08 (C-34), 70.65 (C-32), 68.74 (C-31), 67.74 (C-33), 63.92 (C-29), 62.76 (C-21), 61.92 (C-35), 53.92 (C-26), 51.73 (C-28), 47.27 (C-24), 24.47 (C-25), 21.00 (C-41), 20.96 C-42), 20.87 (C-40), 20.82 (C-43).

### Synthesis of compound 1

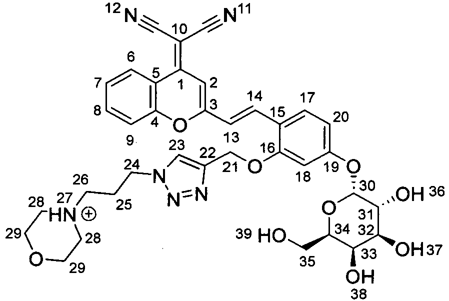

To a chilled solution of compound 2 (42 mg, 0.048 mmol), in dry methanol (4 mL), sodium methoxide (15.5 mg, 0.288 mmol) was added, and the reaction mixture was stirred at room temperature for 6 hours. After the complete consumption of the starting material, the solvent was removed after reduced pressure and the residue was purified by HPLC (98:2 H₂O: ACN to 40: 60 H₂O: ACN, 254 nm) to afford the desired compound as brown solid (21 mg, 62.6 %). ¹H NMR (400 MHz, DMSO-d₆) δ: 9.78 (br, 1H, NH-27), 8.78-8.71 (d, *J* = 8.3 Hz, 1H, H-6), 8.34 (s, 1H, H-23), 7.94-7.88 (t, *J* = 7.6 Hz, 1H, H-8), 7.87-7.82 (d, *J* = 16.2 Hz, 1H, H-14), 7.78-7.72 (d, *J* = 8.7 Hz, 1H, H-17), 7.78-7.72 (d, *J* = 8.4 Hz, 1H, H-9), 7.65-7.58 (t, *J* = 7.4 Hz, 1H, H-7), 7.37-7.30 (d, *J* = 16.0 Hz, 1H, H-13), 7.02-6.99 (d, *J* = 2.1 Hz, 1H, H-18), 6.89 (s, 1H, H-2), 6.76-6.72 (dd, *J* = 8.4 Hz *& J* = 2.1 Hz, 1H, H-20), 5.35 (s, 2H, H-21), 5.28 (br, 1H, OH-36), 5.00 (d, *J* = 7.5 Hz, 1H, H-30), 4.72 (br, 1H, OH-37), 4.62-4.46 (m, 3H, H-24 & H-38), 4.04-3.91 (m, 2H, H-29), 3.74 - 3.50 (m, 7H, H-34, H-33, H-32, H-31, OH-39 & H-29), 3.47-3.41 (m, 2H, H-35), 3.20 - 3.12 (m, 2H, H-26), 3.10- 2.95 (br, 2H, H-28), 2.30-2.19 (q, 2H, H-25). ¹³C NMR (100 MHz, DMSO-d₆) δ: 161.20 (C-3), 159.39 (C-16), 158.49 (C-4), 153.33 (C-19), 152.46 (C-9), 142.96 (C-22), 135.80 (C-8), 134.06 (C-17), 130.42 (C-14), 126.65 (C-7), 125.16 (C-23 & C-6), 119.47 (C-5), 117.97 (C-15), 117.81 (C-13), 117.63 (C-12), 116.49 (C-11), 109.74 (C-20), 106.46 (C-2), 102.45 (C-18), 100.99 (C-30), 76.26 (C-34), 73.86 (C-32), 70.63 (C-31), 68.89 (C-32), 63.92 (C-29), 62.53 (C-21), 60.97 (C-35), 53.95 (C-26), 51.75 (C-28), 47.29 (C-24), 24.49 (C-25).

### Example 2

Scheme 3 shows the synthesis of compound 7, which is a compound according to the present invention.

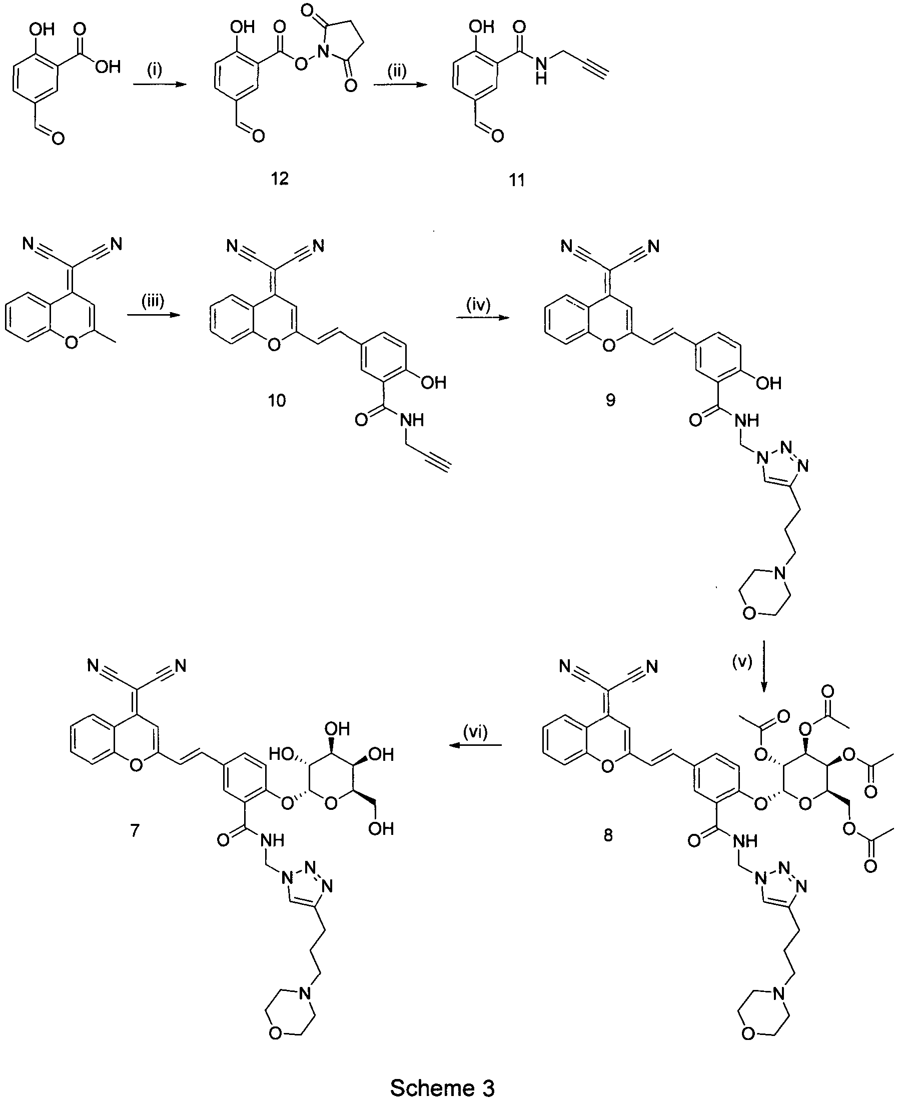

### Synthesis of compound 12

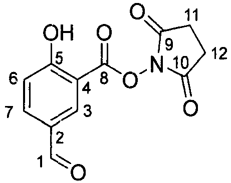

In a solution of 5-formylsalilic acid (500 mg, 3.009 mmol) in anhydrous THF (8 mL) N-Hydroxy succinimide (NHS, 379.2, 3.3 mmol) and N,N'-Dicyclohexylcarbodiimide (DCC, 680 mg, 3.3 mmol) were added and the reaction mixture was stirred at room temperature for 30 minutes. The residue was then filtered to remove the produced Dicyclohexylurea (DCU). The solvent was then removed after reduced pressure to afford a yellowish solid (600 mg, 75.7 %).¹H NMR (250 MHz, CDCl₃): δ 10.15-9.97(d, *J* = 12.2 Hz, 1H, OH-5), 9.96-9.88 (s, 1H, H-1), 9.59-9.50 (d, *J* = 2.1 Hz, 1H, H-3), 8.21-8.09 (dd, *J* = 8.7, 2.1 Hz, 1H, H-7), 7.24-7.16 (d, *J* = 8.7 Hz, 1H, H-6), 3.03-2.88 (s, 5H, H-11, H-12).

### Synthesis of compound 11

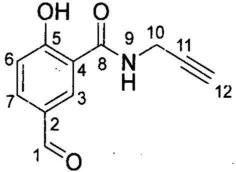

To a solution of compound **12** (500 mg, 1.9 mmol), and triethylamine (TEA, 961.4 mg, 9.5 mmol) in anhydrous ACN (20 mL), propargylamine (209.2, 3.8 mmol) was added and the reaction mixture was stirred at room temperature for 16 hours. Extraction followed with 1N Hydrochloric solution\Ethyl acetate (EA). The organic phase was collected and after concentration with Na₂SO₄, the product was purified by column chromatography 40% EA/Hexane. Then the solvent was removed after reduced pressure to afford the desired compound as white solid (340 mg, 88 %). ¹H NMR (250 MHz, DMSO-d₆): δ 13.56-12.55 (m, 1H, 0H-5), 9.89-9.81 (s, 1H, H-1), 9.45-9.28 (t, *J* = 5.6 Hz, 1H, NH-9), 8.50-8.41 (d, *J* = 2.1 Hz, 1H, H-3), 8.01-7.88 (dd, *J* = 8.6, 2.1 Hz, 1H, H-7), 7.18-7.06 (d, *J* = 8.5 Hz, 1H, H-6), 4.18-4.07 (dd, *J* = 5.5, 2.5 Hz, 2H, H-10), 3.22-3.14 (t, *J* = 2.5 Hz, 1H, H-12).¹³C NMR (63 MHz, DMSO-d₆) δ: 191.30 (C-1), 167.70 (C-8), 164.77 (C-5), 134.81 (C-3), 132.11 (C-2), 128.57 (C-7), 118.69 (C-4), 116.64 (C-6), 80.99 (C-11), 73.78 (C-12), 28.99 (C-10).

### Synthesis of compound 10

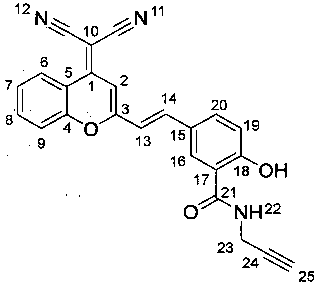

To a solution of compound 2-(2-methyl-4H-chromen-4-ylidene) malononitrile (318 mg, 1.53 mmol) and compound **11** (310 mg, 1.53 mmol) in anhydrous ACN (8 mL) was added a catalytic amount of piperidine and the reaction mixture was refluxed under nitrogen for 14 hours. After the reaction was completed, the solvent was evaporated under reduced pressure and the reaction mixture was purified by column chromatography to afford the desired product as a red solid (340 mg, 56.8%).¹H NMR (400 MHz, DMSO-d₆) δ 13.20-12.09 (br, 1H, OH-18), 9.42-9.28 (s, 1H, NH-22), 8.76-8.67 (dd, *J* = 8.4, 1.4 Hz, 1H, H-6), 8.33-8.22 (d, *J* = 2.2 Hz, 1H, H-16), 7.95-7.88 (ddd, *J* = 8.6, 7.1, 1.5 Hz, 1H, H-8), 7.84-7.78 (dd, *J* = 8.7, 2.2 Hz, 1H, H-19), 7.78-7.73 (dd, *J* = 8.5, 1.3 Hz, 1H, H-9), 7.71-7.64 (d, *J* = 15.9 Hz, 1H, H-14), 7.63-7.57 (ddd, *J* = 8.4, 7.1, 1.3 Hz, 1H, H-7), 7.30-7.23 (d, *J* = 15.9 Hz, 1H, H-13), 7.01-6.95 (d, *J* = 8.6 Hz, 1H, H-20), 6.94-6.90 (s, 1H, H-2), 4.18-4.12 (dd, *J* = 5.3, 2.5 Hz, 2H, H-23), 3.23-3.20 (t, *J* = 2.5 Hz, 1H, H-25).¹³C NMR (101 MHz, DMSO-d₆) δ 168.05 (C-21), 158.93 (C-18), 153.24 (C-3), 152.47 (C-4), 138.86 (C-14), 135.83 (C-20), 133.75 (C-15), 129.75 (C-6), 126.59 (C-5), 125.09 (C-16), 119.42 (C-17), 119.02 (C-13), 117.74 (C-19), 117.58 (C-11, 12), 117.32 (C-8), 116.67 (C-7), 116.45 (C-9), 106.41 (C-2), 81.11 (C-24), 73.90 (C-25), 28.89 (C-23).

### Synthesis of compound 9

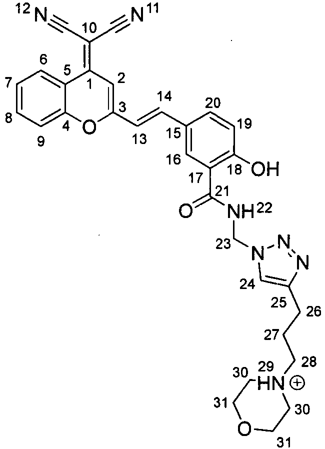

To a solution of compound **10** (150 mg, 0.384 mmol) in DMF (5 mL) under vigorous stirring, sodium ascorbate (15.1 mg, 0.078 mmol mmol) and copper sulfate pentahydrate (7.1 mg, 0.0285 mmol) were added successively. Then followed by the addition of compound 5 (208.4 mg, 0.761 mmol) and the reaction mixture was stirred at room temperature for 16 hours. After the reaction was completed the solvent was removed under reduced pressure and the residue was purified by HPLC chromatography (from 40:70 H₂O: ACN to 0:100 H₂O:ACN, 254 nm) to afford the desired product as a red solid (110 mg, 50.9%).¹H NMR (400 MHz, DMSO-d₆) δ: 13.03-12.73 (br, 1H, OH-32), 10.31-9.23 (br, 1H, NH-22), 9.49-9.37 (t, *J* = 5.7 Hz, 1H, H-8), 8.76-8.66 (dd, *J* = 8.4, 1.5 Hz, 1H, H-6), 8.36-8.26 (d, *J* = 2.3 Hz, 1H, H-16), 8.15-8.05 (s, 1H, H-25), 7.98-7.87 (t, J= 7.4 Hz, 1H, H-7), 7.86-7.78 (dd, *J* = 8.6, 2.1 Hz, 1H, H-19), 7.78-7.71 (d, *J* = 8.4 Hz, 1H, H-9), 7.71-7.64 (d, *J* = 16.0 Hz, 1H, H-14), 7.64-7.56 (t, *J* = 7.8 Hz, 1H, H-20), 7.30-7.22 (d, *J* = 16.0 Hz, 1H, H-13), 7.05-6.98 (d, *J* = 8.7 Hz, 1H, H-2), 6.93-6.88 (s, 1H, NH-29), 4.65-4.58 (d, *J* = 5.4 Hz, 2H, H-23), 4.49-4.41 (t, *J* = 6.9 Hz, 2H, H-26), 4.12-3.84 (br, 2H, H-31), 3.21-2.96 (m, 6H, H-28, H-30), 2.29-2.18 (dq, *J* = 14.5, 7.1 Hz, 2H, H-27).¹³C NMR (101 MHz, DMSO-d₆) δ: 168.47 (C-21), 162.03 (C-18), 158.76 (C-3), 153.31 (C-4), 152.45 (C-24), 144.83 (C-17), 138.65 (C-14), 135.92 (C-25), 134.02 (C-6), 129.12 (C-15), 126.66 (C-16), 126.43 (C-5), 125.11 (C-20), 123.83 (C-19), 119.41 (C-13), 118.73 (C-11, 12), 117.77 (C-8), 117.54 (C-7), 116.42 (C-9), 106.52 (C-2), 63.86 (C-1), 60.31 (C-31), 55.34 (C-30), 53.89 (C-28), 51.70 (C-26), 47.08 (C-23), 24.49 (C-27).

### Synthesis of compound 8

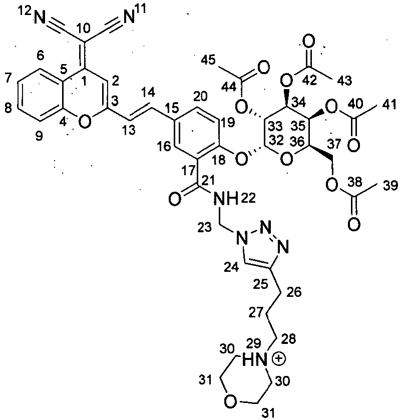

In a chilled solution of compound **9** (87.5 mg, 0.155 mmol) in anhydrous ACN (5 mL), TEA (216.2 µL, 1.55 mmol) was added and the reaction mixture was stirred for 5 min. Tetra-O-acetyl-α-D-galactopyranosyl-1-bromide (254.84 mg, 0.62 mmol) was then added and the reaction mixture was stirred under nitrogen for an additional 15 hours. After the reaction was complete, the solvent was removed under reduced pressure and the residue was purified by HPLC chromatography (from 30:70 H₂O: ACN to 0:100 H₂O:ACN, 254 nm) to obtain the desired product as a brown solid (110 mg, 79.6 %).¹H NMR (400 MHz, DMSO-d₆) δ 9.92-9.66 (br, 1H, NH-22), 8.78-8.72 (d, *J* = 8.3 Hz, 1H, H-6), 8.58-8.51 (t, *J* = 5.8 Hz, 1H, H-8), 8.04-7.86 (m, 4H, H-9, H-14, H-25), 7.78-7.75 (m, 2H, H-7, H-16), 7.67-7.59 (t, *J* = 7.8 Hz, 1H, H-20), 7.55-7.46 (d, *J* = 16.0 Hz, 1H, H-13), 7.30-7.24 (d, *J* = 8.7 Hz, 1H, H-19), 7.08-7.04 (s, 1H, H-2), 5.91-5.86 (d, *J* = 8.3 Hz, 1H, H-36), 5.83-5.79 (d, *J* = 4.9 Hz, 1H, H-32), 5.68-5.62 (d, *J* = 6.0 Hz, 1H, H-35), 5.43-5.26 (m, 5H, H-33, H-34, H-37), 5.12-5.04 (t, *J* = 9.3 Hz, 1H, H-26), 4.58-4.38 (m, 6H, H-23, H-28), 4.25-3.91 (m, 7H, H-31), 3.74-3.57 (m, 4H, H-31), 3.21-2.98 (m, 6H, H-30), 2.31-2.21 (m, 2H, H-27), 2.19-1.91 (m, 12H, H-39, H-41, H-43, H-45).

### Synthesis of compound 7

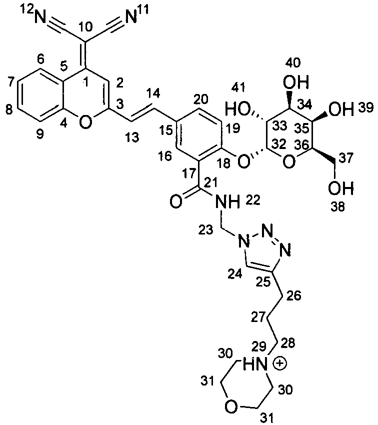

To a chilled solution of compound **8** (87.6 mg, 0.155 mmol) in dry methanol (4 mL), sodium methoxide (43.3 mg, 0.755 mmol) was added, and the reaction mixture was stirred at room temperature for 6 hours. After the reaction was complete, the solvent was removed under high vacuum and the residue was purified by HPLC (from 98:2 H₂O: ACN to 30:70 H₂O:ACN, 254 nm) to afford the desired product as a brown solid (16 mg, 14.3 %).¹H NMR (400 MHz, DMSO-d₆) δ: 9.58-9.39 (br,1H, NH-29), 8.78-8.72 (m, 2H, NH-22, H-8), 8.19-8.16 (d, *J* =1.93 Hz, H-9), 8.04-8.02 (s, 1H, H-25), 7.97 - 7.88 (m, 2H, H-7, H-14), 7.84-7.81 (d, *J* = 3.1 Hz, 1H, H-6), 7.80-7.77 (d, *J* = 4.0 Hz, 1H, H-16), 7.92-7.60 (t, *J* = 7.9 Hz, 1H, H-20), 7.55-7.48 (d, *J* = 16.1 Hz, 1H, H-13), 7.44-7.39 (d, *J* = 8.7 Hz, 1H, H-19), 7.09-7.08 (s, 1H, H-2), 5.07-5.04 (d, *J* = 7.6 Hz, 1H, H-32), 4.68-4.65 (d, *J* = 6.4 Hz, 0H-40), 4.65-4.61 (d, *J* = 5.8 Hz, H-23), 4.58-4.55 (d, *J* = 5.7 Hz, H-37), 4.54-4.51 (d, *J* = 6.2 Hz, OH-41), 4.48-4.43 (t, *J* = 6.8 Hz, 1H, H-26), 4.0-3.93 (d, J=12.7 Hz, 2H, H-28), 3.77-3.74 (m, 1H, H-39), 3.74-3.69 (t, *J* = 6.1 Hz, 1H, H-33), 3.66-3.60 (m, 3H, H-31), 3.59-3.54 (m, 2H, H-27), 3.53-3.51 (m, 1H, OH-38), 3.51-3.49 (m, 1H, H-34), 3.17-3.01 (m, 4H, H-30), 2.28-2.20 (m, 2H, H-35).¹³C NMR (101 MHz, DMSO-d₆) δ: 164.77 (C-21), 158.64 (C-3), 156.96 (C-4), 153.51 (C-18), 152.54 (C-24), 145.74 (C-17), 138.03 (C-16), 135.96 (C-20), 132.47 (C-19), 130.99 (C-14), 129.82 (C-7), 126.70 (C-25), 125.16 (C-6), 124.99 (C-8), 123.40 (C-5), 119.50 (C-15), 117.61 (C-13), 116.32 (C-11, 12), 107.20 (C-2), 102.63 (C-32), 76.49 (C-33), 73.44 (C-35), 70.91 (C-34), 68.65 (C-36), 63.92 (C-31), 60.79 (C-23), 53.96 (C-28), 51.81 (C-30), 47.06 (C-26), 35.57 (C-37), 24.66 (C-27).

### Example 3

Scheme 4 shows the synthesis of compound 13, which is a compound according to the present invention.

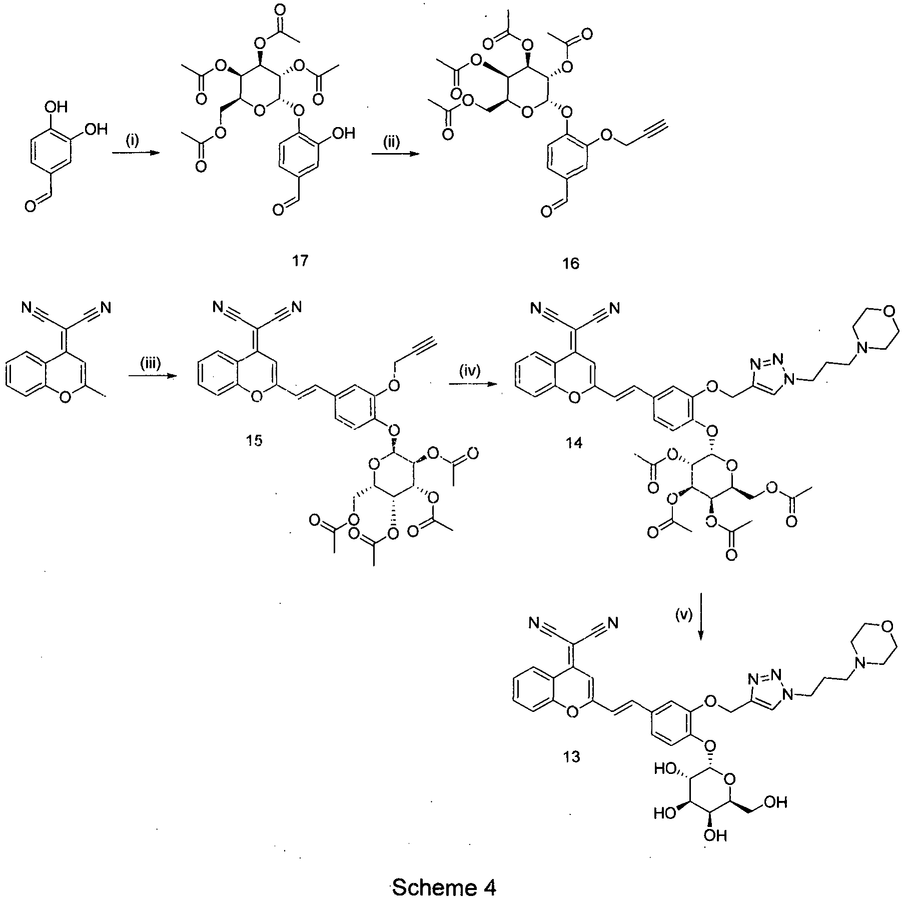

### Synthesis of compound 17

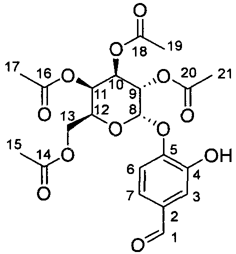

To a solution of compound 3,4-dihydroxy benzaldehyde (50 mg, 0.36 mmol), and anhydrous K₂CO₃ (50 mg, 0.36 mmol) in dry ACN (4 ml) was added tetra-O-acetyl-α-D-galactopyranosyl-1-bromide (148, 0.36 mmol) and the reaction mixture was stirred at room temperatures for the 8 hours. After the reaction is complete, the solvent was removed, and the product was purified by column chromatography 4% Acetone/CH₂Cl₂ to afford a viscous product (120 mg, 71.2 %). ¹H NMR (250 MHz, CDCl₃) δ 9.89-9.86 (s, 1H, H-1), 7.49-7.45 (d, *J* = 1.9 Hz, 1H, H-3), 7.44-7.38 (dd, *J* = 8.3, 2.0 Hz, 1H, H-7), 7.15-7.09 (d, *J* = 8.2 Hz, 1H, H-6), 5.64-5.56 (br, 1H, OH-4), 5.54-5.48 (q, *J* = 3.4 Hz, 2H, H-13), 5.23-5.14 (dd, *J* = 10.5, 3.4 Hz, 1H, H-8), 5.12-5.06 (d, *J* = 7.8 Hz, 1H, H-12), 4.30-4.12 (m, 3H, H-9, H-10, H-11), 2.23-2.19 (s, 4H, H-19), 2.16-2.11 (s, 4H, H-21), 2.11-2.08 (s, 4H, H-17), 2.07-2.03 (s, 4H, H-15).¹³C NMR (63 MHz, CDCl₃) δ 191.13 (C-1), 170.77 (C-18), 170.46 (C-20), 170.16 (C-16), 170.02 (C-14), 148.81 (C-5), 147.24 (C-4), 133.00 (C-2), 123.17 (C-7), 116.38 (C-3), 115.51 (C-6), 100.54 (C-8), 71.52 (C-12), 70.14 (C-10), 69.03 (C-9), 66.62 (C-11), 61.28 (C-13), 20.89 (C-15), 20.60 (C-17,21), 20.53 (C-19).

### Synthesis of compound 16

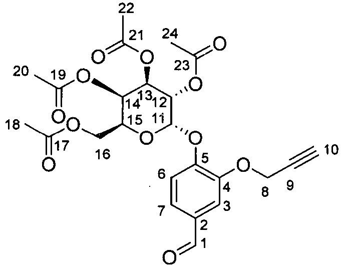

To a solution of compound **17** (121 mg, 0.26 mmol), anhydrous K₂CO₃ (129 mg, 0.93 mmol) in dry ACN (4 mL) was added propargyl bromide (39.9 µL, 0.47 mmol) and the reaction mixture was stirred at room temperature for 16 hours. The solvent was removed under reduced pressure and the product was purified by 3% Acetone/CH₂Cl₂ column chromatography to afford a yellowish oily product (130 mg, 98.7 %). ¹H NMR (250 MHz, CDCl₃) δ 9.92-9.88 (s, 1H, H-1), 7.59-7.54 (d, J=1.8 Hz, 1H, H-3), 7.54-7.46 (dd, *J* = 8.3, 1.8 Hz, 1H, H-7), 7.28-7.23 (d, *J* = 8.3 Hz, 1H, H-6), 5.65 - 5.42 (m, 2H, H-11, H-15), 5.18 - 5.03 (m, 2H, H-16), 4.81-4.75 (t, *J* = 2.5 Hz, 2H, H-8), 4.32 - 4.00 (m, 3H, H-12, H-13, H-14), 2.58-2.52 (t, *J* = 2.4 Hz, 1H, H-10), 2.20-2.16 (s, 4H, H-22), 2.11-2.08 (s, 4H, H-24), 2.08-2.05 (s, 4H, H-20), 2.03-2.00 (s, 4H, H-18).¹³C NMR (63 MHz, CDCl₃) δ 190.63 (C-1), 170.29 (C-21), 170.16 (C-23), 170.08 (C-19), 169.39 (C-17), 152.00 (C-5), 148.47 (C-4), 132.50 (C-2), 126.03 (C-7), 118.04 (C-3), 114.31 (C-6), 100.35 (C-11), 77.71 (C-9), 76.50 (C-10), 71.30 (C-15), 70.54 (C-13), 68.35 (C-112), 66.80 (C-14), 61.31 (C-16), 56.99 (C-8), 20.76 (C-22), 20.62 (C-24, 20), 20.57 (C-18).

### Synthesis of compound 15

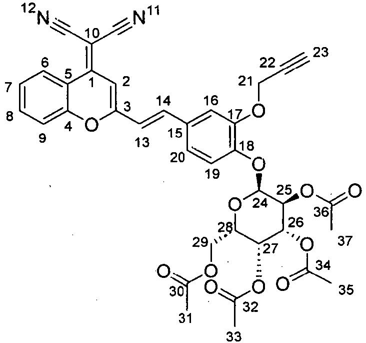

To a solution of compound 2-(2-methyl-4H-chromen-4-ylidene) malononitrile (110 mg, 0.53 mmol) and compound **16** (270 mg, 0.53 mmol) in dry ACN (8 mL) was added a catalytic amount of piperidine and the reaction mixture was refluxed under nitrogen for 14 hours. After the reaction was completed, the solvent was evaporated under reduced pressure and the reaction mixture was purified by HPLC (from 98:2 H₂O: ACN to 30:70 H₂O: ACN, 254 nm) to obtain the desired product at 16 min (118mg, 32 %).¹H NMR (400 MHz, DMSO-d₆) δ: 8.77-8.71 (d, J =8.5 Hz, 1H, H-6), 7.96-7.90 (t, *J* = 7.8 Hz, 1H, H-7), 7.83-7.78 (d, *J* = 8.4 Hz, 1H, H-9), 7.75-7.68 (d, *J* = 16.0 Hz, 1H, H-14), 7.65-7.59 (t, *J* = 7.8 Hz, 1H, H-8), 7.57-7.53 (s, 1H, H-2), 7.47-7.37 (m, 2H, H-13, H-19), 7.24-7.19 (d, *J* = 8.4 Hz, 1H, H-20), 7.01-6.97 (s, 1H, H-16), 5.44-5.41 (m, 1H, H-28), 5.37-5.34 (s, 1H, H-25), 5.29-5.25 (m, 2H, H-29), 4.89-4.85 (d, *J* = 2.2 Hz, 2H, H-21), 4.46-4.40 (t, *J* = 6.2 Hz, 1H, H-24), 4.18-4.08 (m, 2H, H-26, H-27), 3.62-3.59 (t, *J* = 2.2 Hz, 1H, H-23), 2.18-2.15 (s, 3H, H-31), 2.08-2.05 (s, 3H, H-35), 2.05-2.02 (s, 3H, H-37), 1.97-1.94 (s, 3H, H-39).¹³C NMR (100 MHz, DMSO-d₆) δ: 170.46 (C-30), 170.33 (C-34), 170.03 (C-36), 169.57 (C-32), 158.67 (C-3), 153.45 (C-4), 152.53 (C-18), 148.68 (C-17), 147.96 (C-19), 138.68 (C-16), 135.96 (C-8), 130.95 (C-14), 126.68 (C-7), 125.14 (C-9), 123.60 (C-6), 119.53 (C-5), 119.26 (C-15), 118.01 (C-13), 117.59 (C-12), 116.39 (C-11), 114.35 (C-2), 107.00 (C-20), 99.10 (C-24), 79.11 (C-23), 71.02 (C-22), 70.75 (C-25), 68.67 (C-26), 67.71 (C-27), 61.81 (C-28), 60.59 (C-29), 56.87 (C-21), 21.00 (C-31, C-35), 20.88 (C-37), 20.83 (C-33).

### Synthesis of compound 14

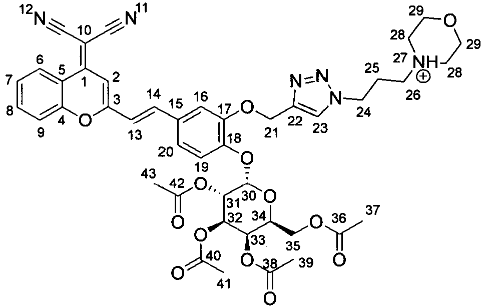

To a solution of compound 15 (118.2 mg, 0.384 mmol) in DMF (4 mL) under vigorous stirring, sodium ascorbate (6.4 mg, 0.032 mmol mmol), copper sulfate pentahydrate (2.96 mg, 0.011 mmol) was added sequentially. Then followed by the addition of compound 5 (110 mg, 0.64 mmol) and the reaction mixture was stirred at room temperature for 16 hours. After the reaction was complete, the solvent was removed under reduced pressure and the residue was purified by HPLC (from 40:70 H₂O: ACN to 0:100 H₂O: ACN, 254 nm) to obtain the desired product as a red solid (68 mg, 20.5 %).¹H NMR (400 MHz, DMSO-d₆) δ: 10.06-9.62 (br, 1H, NH-27), 8.78-8.73 (d, *J* = 8.4 Hz, 1H, H-6), 8.24-8.21 (s, 1H, H-23), 7.98-7.91 (t, *J* = 7.9 Hz, 1H, H-7), 7.83-7.78 (d, *J* = 8.4 Hz, 1H, H-9), 7.78-7.72 (d, *J* = 16.0 Hz, 1H, H-14), 7.71-7.67 (s, 1H, H-16), 7.67-7.60 (t, *J* = 7.8 Hz, 1H, H-8), 7.52-7.43 (d, *J* = 16.0 Hz, 1H, H-13), 7.42-7.36 (d, *J* = 8.4 Hz, 1H, H-19), 7.22-7.17 (d, *J* = 8.4 Hz, 1H, H-20), 7.00-6.98 (s, 1H, H-2), 5.48-5.43 (d, *J* = 7.0 Hz, 1H, H-30), 5.39-5.33 (d, *J* = 2.4 Hz, 1H, H-32), 5.29-5.21 (m, 5H, H-21, H-33), 4.56-4.49 (t, *J* = 6.7 Hz, 2H, H-24), 4.48-4.42 (t, *J* = 6.2 Hz, 1H, H-34), 4.19-4.05 (m, 3H, H-31, H-35), 4.03-3.87 (br, 2H, H-29β), 3.73-3.56 (br, 3H, H-29 α), 3.24-2.95 (m, 6H, H-26, H-29), 2.32-2.23 (m, 2H, H-25), 2.17-2.13 (s, 3H, H-37), 2.06-2.02 (s, 3H, H-39), 1.96-1.93 (s, 3H, H-41), 1.93-1.89 (s, 3H, H-43).¹³C NMR (100 MHz, DMSO-d₆) δ: 170.41 (C-36), 170.33 (C-38), 169.98 (C-40), 169.82 (C-42), 158.76 (C-3), 153.46 (C-4), 152.53 (C-18), 148.78 (C-17), 148.44 (C-19), 143.37 (C-16), 138.86 (C-22), 136.0 (C-8), 130.89 (C-14), 125.18 (C-9), 123.51 (C-23), 119.49 (C-6), 119.10 (C-5), 117.64 (C-15), 117.61 (C-13), 117.23 (C-12), 116.43 (C-11), 113.37 (C-2), 106.96 (C-20), 98.74 (C-30), 71.02 (C-34), 68.72 (C-33), 67.70 (C-32), 63.94 (C-29), 62.85 (C-31), 61.79 (C-21), 60.53 (C-35), 53.92 (C-26), 51.77 (C-28), 47.27 (C-24), 24.56 (C-25), 21.00 (C-43), 20.85 (C-39, C-41), 20.80 (C-37).

### Synthesis of compound 13

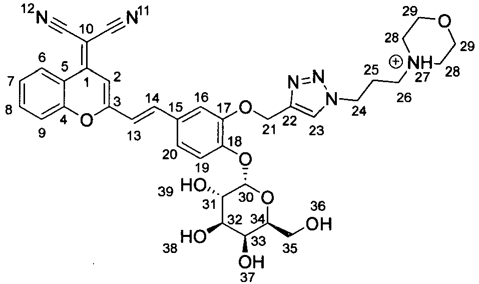

To a chilled solution of compound **14** (65 mg, 0.076 mmol) in dry methanol (4 mL), sodium methoxide (20.9 mg, 0.32 mmol) was added, and the reaction mixture was stirred at room temperature for 6 hours. After the reaction was completed, the solvent was removed under high vacuum and the residue was purified by HPLC (from 98:2 H₂O: ACN to 30:70 H₂O: ACN, 254 nm) to afford the desired product as a brown solid (18 mg, 34 %).¹H NMR (400 MHz, DMSO-d₆) δ: 9.79 (br, 1H, NH-27), 8.78-8.73 (d, *J* = 8.4 Hz, 1H, H-6), 8.36-8.27 (s, 1H, H-23), 7.97-7.91 (t, *J* = 7.8 Hz, 1H, H-7), 7.84-7.77 (d, *J =* 8.4 Hz, 1H, H-9), 7.77-7.69 (d, *J* = 16.0 Hz, 1H, H-14), 7.69-7.59 (m, 2H, H-8, H-16), 7.48-7.39 (d, *J =* 16.0 Hz, 1H, H-13), 7.37-7.31 (d, *J* = 8.3 Hz, 1H, H-19), 7.24-7.18 (d, *J* = 8.4 Hz, 1H, H-20), 7.01-6.96 (s, 1H, H-2), 5.32 (s, 2H, H-21), 5.21-5.06 (br, 1H, OH-36), 5.04 (d, *J* = 7.8 Hz, 1H, H-30), 4.81-4.59 (br, 2H, OH-37, H-37), 4.53-4.46 (t, *J =* 6.6 Hz, 2H, H-24), 4.06-3.89 (m, 2H, H-35), 3.78-3.51 (m, 7H, H-29, H-31, H-33, H-39), 3.22- 2.98 (m, 4H, H-26, H-28), 2.31-2.22 (q, 2H, H-25). ¹³C NMR (100 MHz, DMSO-d₆) δ: 158.98 (C-3), 152.55 (C-4), 149.81 (C-9), 139.22 (C-17, C-18), 135.95 (C-22), 129.49 (C-8, C-18), 126.69 (C-14), 125.26 (C-7), 124.05 (C-6, C-23), 119.51 (C-5), 118.37 (C-15), 117.72 (C-11, C-12), 116.48 (C-20), 114.22 (C-2), 106.70 (C-16, C-19), 100.96 (C-30), 76.07 (C-34), 73.89 (C-32), 70.69 (C-31), 68.56 (C-33), 63.91 (C-29), 63.13 (C-21), 63.80 (C-35), 53.91 (C-26), 51.76 (C-28), 47.20 (C-24), 29.48 (C-25).

### Example 4

### Spectral properties and selectivity of compound 1

To monitor the responsiveness of compound 1 (Example 1) towards *β*-galactosidase, UV-Vis and fluorescence studies were performed. Compound 1 in DMSO: PB (3:7, 10 mM pH 6.00) displays a distinct absorption spectrum with λₘₐₓ centered at 425 nm. Upon the gradual addition of *β*-galactosidase (up to 200 mU), a distinct 160 nm bathochromic shifting was observed, accompanied by the presence of one isosbestic point centered at 465 nm. This finding could be explained by the hydrolysis of the sugar moiety attached in the phenolic group of compound 1 resulting in its transformation into compound 3.

Furthermore, the fluorescent responsiveness of compound 1 in the presence of *β-*galactosidase upon excitation at 465 and 585 nm was evaluated. With the gradual addition of *β*-galactosidase (0-200 mU) upon excitation at the isosbestic point of the absorbance spectra the ratiometric fluorescent behavior (I₆₉₀/I₅₀₀) of the probe was recorded. As illustrated in Figure 1, the enhancement of the fluorescent signal was significant accompanied by a remarkable bathochromic shifting into the emission range of 550-850 nm along with a decrease in the emission band at 500 nm. More specifically, the ratiometric fluorescent signal (I₆₉₀/I₅₀₀) was significantly 8-fold increased. Moreover, a district 20-fold enhancement at 690 nm was observed upon excitation at 585 nm. This finding could be explained by the *β*-galactosidase mediated hydrolysis of the sugar moiety that releases compound 3 restoring in this way the ICT process. Next, the fluorescent spectra of compound 1 as function of time in presence of 100 mU of the enzyme waw recorded. As illustrated in Figure 2, the fluorescent signal gradually increased upon excitation at 465 and 585 nm. The ratiometric fluorescent signal (I₆₉₀/I₅₀₀) as well as the fluorescent signal at I₆₉₀ displayed good linear relationship in this set of experiments.

To exploit the response rate of compound 1 in the presence of *β*-galactosidase, time-course fluorescent experiments were conducted, by monitoring the fluorescence signal at 690 nm in the presence of different concentrations of the enzyme. As illustrated in Figure 3A in the presence of 0.5 U and 1.0 U of the enzyme the fluorescent signal of the probe reaches its maximum value within 240 sec and 480 sec, respectively. The detection limit was calculated at 0.49 mU, indicating that compound 1 has enhanced sensitivity to changes of *β*-gal concentrations. The selectivity of this probe towards different analytes including metals, aminoacids and Reactive Oxygen Species (ROS) was further evaluated *via* fluorescence spectroscopy by monitoring the ratiometric fluorescent signal (I₆₉₀/I₅₀₀). As illustrated in Figure 3B, the ratiometric fluorescent signal was barely altered by the addition of any analytes (100 µM) [except *β*-galactosidase (200 mU)], indicating the enhanced selectivity of our probe.

### Example 5

### Biological evaluation of compound 1

### Cytotoxicity

The effect of compound **1** (Example 1) on the viability of BJ fibroblasts (MTT assay) was evaluated. Cells were incubated with increasing concentrations of compound **1** for 24 and 48h (Figure 4). It was found that the probe did not exert any cytotoxic effect in human normal fibroblasts.

### Imaging of endogenous β-gal in live cells

It was examined whether compound **1** can be used to track and visualize *β*-gal activity in senescent cells. Thus, premature senescence (SIPS) was triggered by exposing young proliferating BJ fibroblasts to the oxidative agent hydrogen peroxide (H₂O₂). H₂O₂ is the most widespread used agent to induce SIPS in normal cells leading to the overexpression of SA-*β*-gal (Ott, C., Jung, T., Grune, T. & Höhn, A. SIPS as a model to study age-related changes in proteolysis and aggregate formation. Mechanisms of ageing and development 170, 72-81, doi:10.1016/j.mad.2017.07.007 (2018)). Herein, three exposures were performed (48 h each) of cells to 300 µM H₂O₂ and, as shown in Figure 5A, 80±7 % of cells were stained positive in SA-*β*-gal staining post- H₂O₂ treatment. Normal untreated BJ cells were used as control group. Both control and H₂O₂-treated BJ cells were incubated with compound **1** (10 µM) in culture medium for 1h at 37 °C and were then observed with a Digital Eclipse C1 Nikon confocal laser scanning microscope (CLSM) upon excitation of the probe at 543 nm. As shown in Figure 5B, higher levels of fluorescence was observed in senescent cells, while aberrantly minimal fluorescence seen in young replicating (control) cells. As a negative control, both control and senescent cells were pretreated with the *β*-gal competitive inhibitor, namely D-galactose (1 mM) for 1 h and were then incubated with the compound **1** (10 µM) for 1h at 37 °C. Notably, the senescence-associated increased fluorescence signal produced in senescent cells by compound **1** was barely observed after preincubation with D-galactose (Figure 5B), further supporting the capability of the probe to sense *β*-gal activity specifically in senescent cells.

### Ex vivo imaging

Given the fact that compound **1** selectively visualizes *β*-gal activity in senescent cells, its activity *ex vivo* was investigated. To this point, three main organs, namely heart, liver, and kidney from 5- and 24-month-old C57BL/6J male mice, were isolated and sliced (see Materials and Methods). The tissue sections were treated with compound 1 for 1 h and observed by CLSM. As illustrated in Figure 6, it was found that fluorescence intensity was increased significantly in all tissues from 24-months old mice, with the fluorescence levels in aged heart and kidneys being six times higher than those seen in these organs when derived from young mice.

### Example 6

### Spectral properties of compound 7

Compound **7** (Example 2) is similar to compound **1,** with the difference that the targeting group is linked by an amide bond, instead of an ether bond. The enzyme's type of action and the trigger group remain the same.

To observe the reactivity of compound **7** to *β*-galactosidase, fluorescence experiments were carried out. Upon excitation at 475 and 585 nm, compound 7 was analyzed in DMSO: PB (3:7, 10 mM pH 6.00) in the presence of *β*-galactosidase. Upon the gradual addition of *β*-galactosidase (0-400 mU) at 475 nm excitation, there is a shift from 690 nm to 705 nm; also, an 60-fold enhancement at 690 nm was observed. Upon excitation at 585 nm an 70-fold enhancement at 690 nm was noticed. This finding could be potentially explained by the *β*-galactosidase mediated hydrolysis of the sugar moiety (that releases compound **9)** restoring in this way the ICT process. After that the fluorescence spectra of compound **7** (against 100 mU of the enzyme) as a function of time were recorded. Following stimulation at 475 and 585 nm, the fluorescent signal gradually increased approximately 20 times. In these studies, the fluorescent signal at I₇₀₅ and at I₆₉₀ showed good linear relationships.

By observing the fluorescence signal at 690 nm upon excitation at 585 nm, time-course fluorescent studies were performed to take advantage of the response rate of compound **7** in the presence of various concentrations of the enzyme. As the enzyme concentration is raised to 1.0 U and 2.0 U, the fluorescence signal of the probe rises almost linearly. Fluorescence spectroscopy was used to further assess the probe's selectivity for various analytes, such as metals, amino acids, and ROS, by keeping track of the ratiometric fluorescent signal (I₇₀₅/I₅₅₀). The addition of many analytes (100 µM) other than *β-*galactosidase (400 mU) minimally changed the ratiometric fluorescent signal, demonstrating the enhanced selectivity of our probe.

### Example 7

### Biological evaluation of compound 7

### Cytotoxicity

Cytotoxicity of compound **7** was examined on BJ fibroblasts by performing the MTT assay with increasing concentrations of compound **7** for 24 and 48h. As shown in Figure 7, no significant toxicity in human normal fibroblasts was found.

### Imaging of endogenous β-gal in live cells

Compound **7** was also evaluated for its ability to visualize *β*-gal specifically in senescent cells. Following the same procedure as for compound 1, a model of premature senescent (SIPS) induction in cells was created by three continuous exposures of young replicating BJ fibroblasts to H₂O₂. Normal untreated and H₂O₂-treated BJ cells were incubated with 10 µM of compound **7** in culture medium for 1h at 37 °C. Cells treated with 1 mM of D-galactose were used also as a negative control. The analysis showed that senescent (H₂O₂ treated) cells (Figure 8A) exhibited significantly higher levels of staining intensity (CLSM) after exposure to compound **7** in comparison with control and cells treated with D-galactose (Figure 8B).

### Ex vivo imaging

Compound 7 was tested for its activity *ex vivo.* As mentioned before (Example 5), sections from three main organs, namely heart, liver, and kidney from 5- and 24-month-old C57BL/6J male mice, were treated with compound 7 for 1h and evaluated by CLSM. The findings in cells were verified also in tissues since older sliced tissues illustrated more intense signal than younger controls. (Figure 9).

### Example 8

### Spectral properties and selectivity of compound 13

Compounds **7** and **1** are structurally very similar. Both targeting groups are connected through an ether bond. The difference in compound **13** lies in the position of the etheric bond related to the main core of the dye.

To determine compound's **13** *β*-gal selectivity, similar fluorescence experiments to the two previous cases (described above for compounds **1** and **7)** were conducted. Upon excitation at 465 and 585 nm, compound **13** was analyzed in DMSO: PB (3:7, 10 mM pH 6.00) in the presence of *β*-galactosidase. Upon the gradual addition of *β-*galactosidase (0-400 mU) and excitation at 465 nm, there is a shift from 572 to730 nm. Also, a district 12-fold enhancement at 730 nm was observed. Upon excitation at 585 nm a 9-fold enhancement at 730 nm was noticed. This finding could be explained by the *β*-galactosidase mediated hydrolysis of the sugar moiety restoring in this way the ICT process. Then, with 150 mU of the enzyme present, compound **13'**s fluorescence spectra was recorded as a function of time; a steadily increased fluorescent signal was noted after stimulation at 465 and 585 nm. The fluorescent signal at I₇₃₀ in this study displayed efficient linear correlations.

To observe the response rate of compound **13** in the presence of *β*-galactosidase; kinetic fluorescent experiments were conducted by monitoring the fluorescence signal at 730 nm at different concentrations of the enzyme. In the presence of 2.0 U the fluorescent signal of the probe achieves its peak value in 400 seconds. The selectivity of this probe towards different analytes including metals, amino acids and ROS was further evaluated *via* fluorescence spectroscopy by monitoring the ratiometric fluorescent signal (I₇₃₀/I₅₃₅). The ratiometric fluorescent signal was barely altered by the addition of any analytes (100 µM) but *β*-galactosidase (400 mU), demonstrating the great selectivity of our probe.

### Example 9

### Biological evaluation of compound 13

### Imaging of endogenous β-gal in live cells

It was investigated whether compound **13** possesses the ability to visualize *β*-gal in senescent cells as compounds **1** and **7,** in a model of premature senescence. On that purpose normal untreated and H₂O₂-treated BJ cells were incubated with 5 µM of compound **13** and 1 mM of D-galactose in culture medium for 1h at 37 °C. As illustrated in Figure 10, compound **13** staining showed a significant increase in H₂O₂-treated BJ cells.

### Ex vivo imaging

Compound **13** was assessed, as compounds **1** and **7,** in sections from three main organs, i.e., heart, liver, and kidney from 5- and 24-month-old C57BL/6J male mice. The findings, as shown in Figure 11, confirmed its capacity to stain *β*-gal in aged tissues, as sections from 24- months mice displayed higher levels of fluorescence in CSLM.

## Claims

1. A compound of Formula (1) or a salt thereof 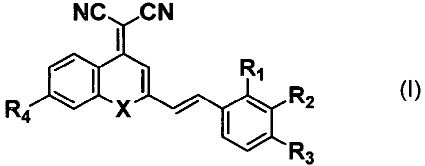 wherein
- X is selected from the group consisting of O, S, Se, -N-(CH₂)ₙ-CH₃;
- each one of R₁, R₂ and R₃ is selected from the group consisting of
H, halogen, 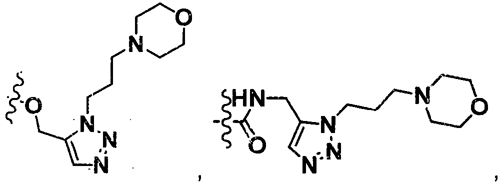 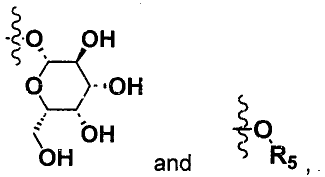 provided that
a) one of R₁, R₂ and R₃ is 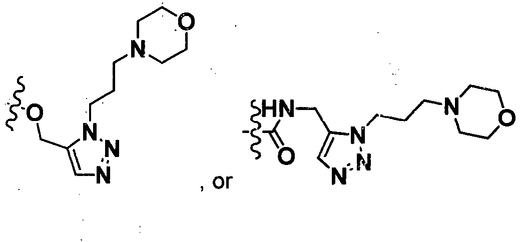 and
b) one of R₁, R₂ and R₃ is 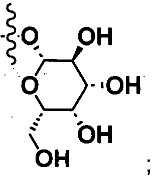
- R₄ is H, or 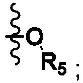
- R₅ is H, or -(CH₂)ₙ-CH₃;
- n is 0, 1, 2, or 3.

2. The compound or a salt thereof according to claim 1, wherein X is selected from the group consisting of O, S and Se.

3. The compound or a salt thereof according to claim 1 or 2, wherein R₁ or R₃ is

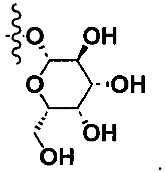

4. The compound or a salt thereof according to any one of the preceding claims, wherein
- R₃ is 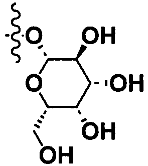 and
- X is selected from the group consisting of O, S and Se.

5. The compound or a salt therefor according to any one of claims 1 to 3, wherein
- R₁ is 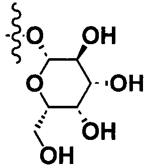 and
- X is selected from the group consisting of O, S and Se.

6. The compound or a salt thereof according to any one of the preceding claims, wherein the halogen is selected from the group consisting of Cl, Br and I.

7. The compound or a salt thereof according to any one of the preceding claims, wherein the salt is selected from the group consisting of sodium, potassium, calcium, magnesium, ammonium, choline, diethylamine, 2-diethylaminoethanol, N,N- dimethylethanolamine, tromethamine, ethanolamine, lysine and arginine salts.

8. Use of the compound or a salt thereof according to any one of the preceding claims for the detection of senescent cells in a biological sample.

9. The use according to claim 8, wherein the biological sample is obtained from a human subject.

10. The use according to claim 8 or 9, wherein the detection is performed *in vitro* or *ex vivo.*
